# EUROPEAN PATENT SPECIFICATION

(11) **EP 0 257 942 B1**
(45) Date of publication and mention of the grant of the patent: **16.03.1994**
(21) Application number: 87307219.3
(22) Date of filing: 14.08.1987
(51) Int. Cl.: A61L 2/18, G02C 13/00

(54) **Use of a cleaning composition for contact lens cleaning**
Verwendung eines Reinigungssystems für die Reinigung von Kontaktlinsen
Utilisation d'une composition de nettoyage pour le nettoyage des lentilles de contact

(30) Priority: 15.08.1986 JP 191520/86
(43) Date of publication of application: 02.03.1988
(73) Proprietor: HOYA CORPORATION, Shinjuku-ku Tokyo (JP)
(72) Inventor: Itagaki, Yoko, Kodama-gun Saitama-ken (JP); Hiranuma, Masahiro, Honjo-shi (JP)
(74) Representative: Darby, David Thomas

(56) References cited:
- EP-A- 0 139 994
- EP-A- 0 147 100
- EP-A- 0 175 801
- EP-A- 0 196 075
- FR-A- 2 256 767
- FR-A- 2 544 880

## Description

The present invention relates to the use of a package for contact lens cleaning. The present invention is especially applicable to cleaning of known contact lenses composed mainly of, for example, polymers of methyl methacrylate, silicone-containing methacrylate, hydroxyethyl methacrylate, butyl acrylate or the like.

Methods for cleaning contact lenses to remove stains adhering thereto are already known and various compositions have been proposed for this purpose.

For example, Japanese Patent Application Kokai (Laid-Open) No. 42614/1982 discloses a method wherein a contact lens is immersed in an aqueous solution containing, for example, a hydrochlorite, a chlorinated lime and chloramine to clean the lens. This method has a sufficient cleaning effect but pays no attention to making the cleaned lens safe by, for example, applying to the cleaned lens a treatment for making it nontoxic. Japanese Patent Application Kokai (Laid-Open) No. 119113/1981 discloses a method wherein a contact lens is immersed in an aqueous solution containing a hypohalogenite for a length of time necessary to remove any stains adhering to the lens. In this method, a reducing agent is dissolved in the cleaning solution, after the immersion treatment for the necessary length of time, to make the solution nontoxic and, when the reducing agent is a saccharide such as glucose, mannose or the like, it can be dissolved in the cleaning solution prior to the immersion. These methods, however, are defective in that the treatment for making the solution nontoxic may be forgotten and the visual check of the completion timing of such a treatment is difficult.

FR-A-2 256 762 discloses a procedure for sterilising contact lenses using an oxidising agent and a reducing agent. Calcium hypochlorite and ascorbic acid are among the compounds listed as being suitable for use as the oxidising agent and the reducing agent, respectively. The oxidising agent and reducing agent may be formulated as a single tablet with the reducing agent being coated by a protective layer.

Thus, the conventional methods for removing stains from contact lenses have a sufficient stain-removing effect but have drawbacks, for example, in that the safety of the cleaned lens is low, that the cleaning operation becomes lengthy because two cleaning components are added separately, that the addition of one cleaning component is forgotten resulting in the reduced safety of the cleaned lens, and that the safety of the cleaned lens cannot be ascertained.

It is an object of the present invention to provide the use of a package for contact lens cleaning which can remove stains adhering to contact lenses effectively in a short time, and with which, after stain removal, a treatment may be reliably conducted for making the cleaned lenses nontoxic, and which enables the user easily and visually to check the completion of said treatment for making the cleaned lenses nontoxic.

Another object of the present invention is to provide the use of a package which can effect both stain removal and a treatment for making the cleaned lens nontoxic in only a single cleaning operation and makes completely safe the possible wearing of the resulting lens right after the operation without the need for washing the lens with tap water, purified water or running water.

Still another object of the present invention is to provide the use of a package which gives no adverse effect on the shape, color tone, etc. of the contact lens even when the lens is subjected to repeated cleaning, and accordingly, which is able to carry out cleaning safely and reliably.

The objects of the present invention can be achieved by the use of a package, as specified below, for contact lens cleaning.

The present invention provides the use, for cleaning without discoloring a contact lens, of a package containing an oxidising agent comprising chlorinated lime powder for stain-removal and a tablet, the contents of the tablet comprising (i) sodium or potassium bicarbonate and (ii) a reducing agent comprising citric acid or citric acid and sodium citrate, the tablet being so coated that the powder does not substantially react with the contents of the tablet in the package and that when the tablet and the powder are placed in water substantially simultaneously the major portion of the chlorinated lime dissolves more rapidly than the major portion of component (ii), whereby, on dissolution of the tablet coating in the water, effervescence takes place and residual chlorinated lime is reduced by component (ii) to render it nontoxic.

The present invention will now be described in detail.

The present invention involves the use of a package containing an oxidizing agent and a reducing agent.

The oxidizing agent, which is a first essential component for contact lens cleaning according to the present invention, is to remove stains adhering to contact lenses. The oxidizing agent comprises a compound capable of releasing available chlorine, namely, chlorinated lime, in the form of a powder.

The reducing agent, which is a second essential component for contact lens cleaning according to the present invention, is to make nontoxic the oxidizing agent still remaining after stain removal. The reducing agent is a hydroxycarboxylic acid, namely, citric acid, used either alone, or, in combination with sodium citrate. The reducing agent is provided in tablet form.

The package has no particular restriction as long as it is made of a material giving substantially no adverse effect on the oxidizing agent and the reducing agent. Typical examples of the package include an aluminum pouch, a three sides-sealed aluminum package and a stick-shaped aluminum package.

The oxidizing agent and the reducing agent are in forms that satisfy both of the following requirements (I) and (II).
(I) The oxidizing agent and the reducing agent do not substantially react with each other in the package.
(II) When the package is opened and each component is placed in water, the major portion of the oxidizing agent dissolves in the water more rapidly than the major portion of the reducing agent.

In the requirement (I), "do not substantially react with each other" implies that the oxidizing agent and the reducing agent do not react at all, or, do not react significantly with each other while they are contained in the package, so that they can achieve their intended purposes (a cleaning treatment of lens in the case of the oxidizing agent and a treatment of the residual oxidizing agent for making it nontoxic in the case of the reducing agent) when the package is opened and each component is placed in water. Accordingly, even if a very small portion of the oxidizing agent and a very small portion of the reducing agent make mutual contact in the package to cause an extremely low level of reaction, it is not a problem as long as the residual unreacted oxidizing agent and the residual unreacted reducing agent can each achieve their respective intended purposes when they are placed in water.

In the requirement (II), "the major portion of the oxidizing agent dissolves in water more rapidly than the major portion of the reducing agent" implies that, when they are placed in water substantially simultaneously, the dissolution peak of the oxidizing agent appears earlier than the dissolution peak of the reducing agent. Accordingly, even if both the dissolution of the oxidizing agent and the dissolution of the reducing agent start simultaneously at a certain time after they have been placed in water, or even if some of the reducing agent initially dissolves rather quickly, even before some of the oxidizing agent, but the remainder continues to dissolve steadily for a significant period of time after the oxidizing agent has all dissolved, it is not a problem as long as macroscopically the cleaning treatment of the contact lens by the oxidizing agent takes places first and the treatment of the residual oxidizing agent by the reducing agent for making the residual oxidizing agent nontoxic takes place subsequently.

In order for the oxidizing agent and the reducing agent to satisfy the requirements (I) and (II) above, the oxidizing agent is in the form of a powder and the reducing agent is in the form of a tablet. When the oxidizing agent and the reducing agent are each in said form, as compared with when both of them are in the form of powders or granules, they have a low degree of mutual contact and do not substantially react with each other, and, when they are placed in water, the oxidizing agent in powder form dissolves in the water more rapidly than the reducing agent in tablet form.

When using the oxidizing agent in the form of a powder, it is possible to add a filler such as NaCl, KCl, lactose and/or dextrose or the like in order to make measuring easier.

The reducing agent is used in the form of tablets that are coated with a coating agent in order to prevent a direct reaction between the oxidizing agent and the reducing agent in the package and also so that, when they are placed in water, the reducing agent starts to dissolve after the oxidizing agent has substantially achieved its purpose, in order to render the residual oxidizing agent nontoxic, so that,the simultaneous occurrence of (a) lens cleaning by the oxidizing agent and (b) a treatment of the residual oxidizing agent by the reducing agent can be minimized and the two components can be utilized efficiently.

As the coating agent, there are preferred, for example, carboxymethylethylcellulose, cellulose acetate phthalate, hydroxypropylmethylcellulose phthalate, a methacrylic acid-ethyl acrylate copolymer and a methacrylic acid-methyl acrylate copolymer. Any coating agent can be used as long as it is soluble in aqueous solutions having a pH of 5.5 or above.

The tablet containing the reducing agent is also made effervescent in order to ensure that the treatment of the residual oxidizing agent to make it nontoxic is conducted satisfactorily. The component added to make the tablets effervescent may be either sodium bicarbonate or potassium bicarbonate, and those components will achieve the purpose of foaming. The tablets may further contain, for example, a lubricant, or a disintegrating agent, ordinarily used in tablet preparation.

The cleaning of contact lenses, in accordance with the present invention, will now be described. The concentration of the oxidizing agent, chlorinated lime, after dissolution in water is preferred to be 0.005 to 5.0% by w/v.

When using an oxidizing agent that is a bleaching powder which is a compound capable of releasing available chlorine, the concentration of the bleaching powder during the cleaning treatment is suitably 0.01% to 1.0% by w/v after dissolution in water. When the concentration is lower than 0.01% by w/v, the effect of the bleaching powder is small and the treatment takes a long time. When the concentration is higher than 1.0% by w/v, no corresponding increase in effect is obtained. A more preferable concentration of the bleaching powder is 0.1% to 0.5% by w/v after dissolution in water.

The concentration of the reducing agent during use must be such that it provides an amount sufficient for the reduction of the residual oxidizing agent and yet gives no significant effect on the pH and osmotic pressure of the solution after treatment. The concentration of the reducing agent, which will either comprise citric acid or citric acid sodium citrate, after dissolution in water is preferred to be 0.05% to 10.0% by w/v. When the reducing agent is, for example,citric acid alone, its concentration after dissolution in water is preferably 0.1% to 2.0% by w/v, more preferably 0.2% to 1.0% by w/v.

Based on these concentrations after dissolution in water, the amounts of the oxidizing agent and the reducing agent in the package may be determined.

Preferably, the package will contain a predetermined quantity of each of the oxidizing and reducing agents, with instructions to dissolve the contents of the package in water, especially in a specified quantity of water. The instructions may also include directions that the contact lens or lenses should already be in the water or added within a specified time after the package contents.

According to the present invention, when the oxidizing agent and the reducing agent are placed in water substantially simultaneously, the major portion of the oxidizing agent dissolves in water more rapidly than the major portion of the reducing agent because the oxidizing agent and the reducing agent take the above mentioned forms.

Thus, after the oxidizing agent has achieved its purpose of removing stains adhering to a contact lens, the reducing agent treats the residual oxidizing agent to make it nontoxic. This treatment of the residual oxidizing agent by the reducing agent to make the former nontoxic is complete when the reducing agent has dissolved completely. Because the reducing agent is in a tablet , the timing of complete dissolution of the reducing agent can be confirmed definitely by visually checking the complete disappearance of the tablet .

As the reducing agent is in a coated tablet, firstly the oxidizing agent dissolves and starts cleaning of the contact lens , and simultaneously, the coating agent on the tablet begins to dissolve slowly; in 5 to 30 minutes, the reducing agent present in the effervescent tablet begins to dissolve slowly while foaming, and starts a reaction with the residual oxidizing agent to make it nontoxic. Thus, the cleaning treatment of the lens by the oxidizing agent and the treatment of the residual oxidizing agent by the reducing agent for making the residual oxidizing agent nontoxic are conducted in different time spans and this is particularly preferable.

The present invention may be used to clean all known contact lenses. It is desired, however, that the solution after treatment be isotonic with,and have the same pH as,the human lacrima in view of (a) the fact that, in particular, hydrogel contact lenses composed mainly of poly (hydroxyethyl methacrylate) or the like may undergo a change in shape due to the osmotic pressure, pH, etc. of the treating solution used and (b) the compatibility of the cleaned contact lens with the eye.

For this purpose, it is possible to add to the oxidizing agent and the reducing agent isotonicity agents, for example NaCl, KCl, or glucose, and buffer agents, for example, boric acid, sodium borate, sodium hydrogen phosphate, sodium acetate, and sodium carbonate and others known per se. The before-mentioned hydroxycarboxylic acid or salt thereof and foaming agent may be also used as pH-adjusting agent.

The package may be used in the following manner periodically, for example, weekly, biweekly or monthly. That is, a contact lens is removed from the eye, washed with water gently, and then immersed for 30 to 60 minutes in a container containing a required volume of water (tap water or purified water) to which one package, as described above, has been added. In the early period of the immersion, the stains adhering to the contact lens are removed by the oxidizing agent; then the reducing agent dissolves slowly and completes treatment by making the residual oxidizing agent nontoxic. The completion of the treatment of the residual oxidizing agent by the reducing agent for making the residual oxidizing agent nontoxic can be clearly confirmed visually by the disappearance of tablets in treating solution. When the treatment is over, the lens is gently washed with water and then worn or stored in a special preservative solution. If the lens is worn directly after the treatment without water washing, it is safe because there is no damage to eyes.

Repeated cleaning of a contact lens according to the present invention has no effect on the shape, color tone, etc. of the lens. Accordingly, the use of the package is very safe also from this aspect.

The present invention will be explained in further detail by way of Examples.

### Example 1

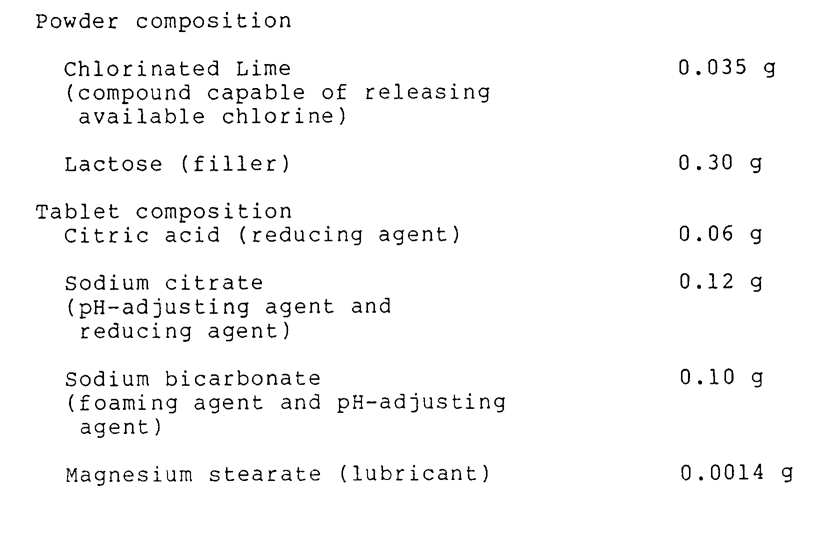

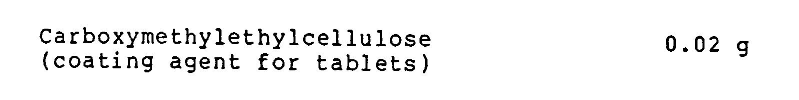

Powders having the above composition and tablets coated with a coating agent having the above composition were prepared according to ordinary methods. They were packed in a three sides-sealed aluminum package. The package was opened and the contents was placed in 10 ml of a tap water. Immediately, an oxygen-permeable hard contact lens (Hoya Hard/OP) having stains as a result of actual wearing was immersed in the solution. In about 15 minutes after the powder had dissolved, the tablets began to dissolve with foaming. In about 60 minutes, the tablets completed dissolution. The lens was taken out from the solution and washed with tap water gently, then its surface was observed using a stereoscopic microscope of 20 magnification manufactured by Neitz. The stains had been removed completely. The solution after treatment had a pH of 7.1 and an osmotic pressure of 350 m0sm.

### Example 2

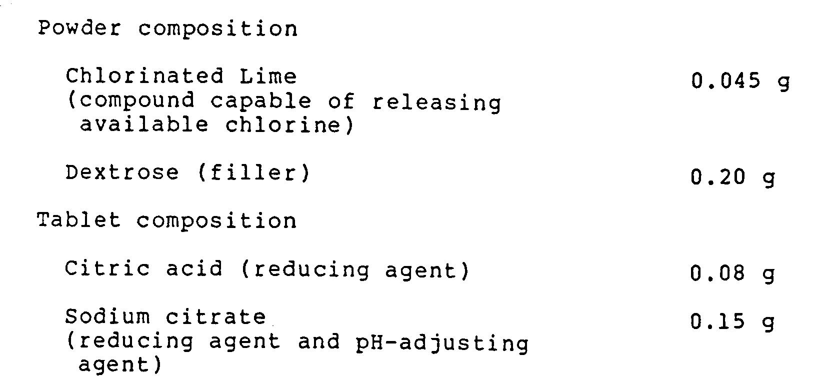

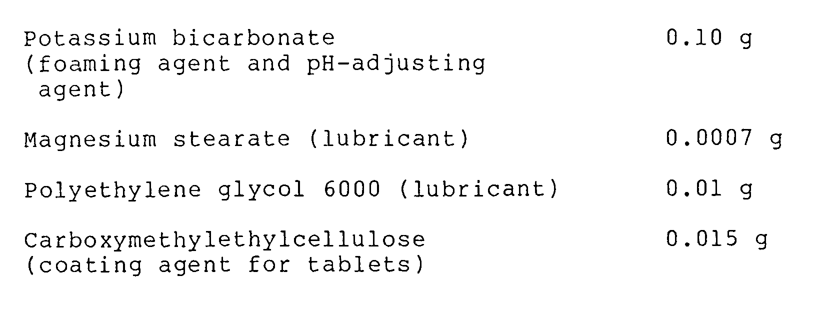

Powders having the above composition and tablets coated with a coating agent, having the above composition, were prepared according to ordinary methods. They were packed in a stick-shaped aluminum package. The package was opened and the contents was placed in 10 ml of purified water. Immediately, a soft contact lens (Hoya Soft) having stains as a result of actual wearing was immersed in the solvent. In about 5 minutes after the powder had dissolved, the tablets began to dissolve with foaming. In about 30 minutes, the tablets completed dissolution. The lens was taken out of the solution and washed with purified water gently. After the water on the lens surface had been wiped off, the lens surface was observed using a stereoscopic microscope of 20 magnification manufactured by Neitz. The stains had been removed completely. The solution after treatment had a pH of 7.3 and an osmotic pressure of 310 m0sm.

Contact lenses (Hoya Soft, Hoya Soft/T40, Hoya Hard and Hoya Hard/OP) were subjected to repeated treatments using the kits of Examples 1 and 2, whereby the effects of these treatments on the shapes of said contact lenses were examined. The results are shown in Table 1. Further, a hard contact lens (Hoya Hard), an oxygen-permeable hard contact lens (Menicon O₂ ® , manufactured by Toyo Contact Lens Co.) and a highly oxygen-permeable hard contact lens (Menicon EX ® , manufactured by Toyo Contact Lens Co.) were subjected to the same repeated treatments, whereby the effects of the treatments on the discoloration of these lenses were examined. The results are shown in Tables 1 and 2.

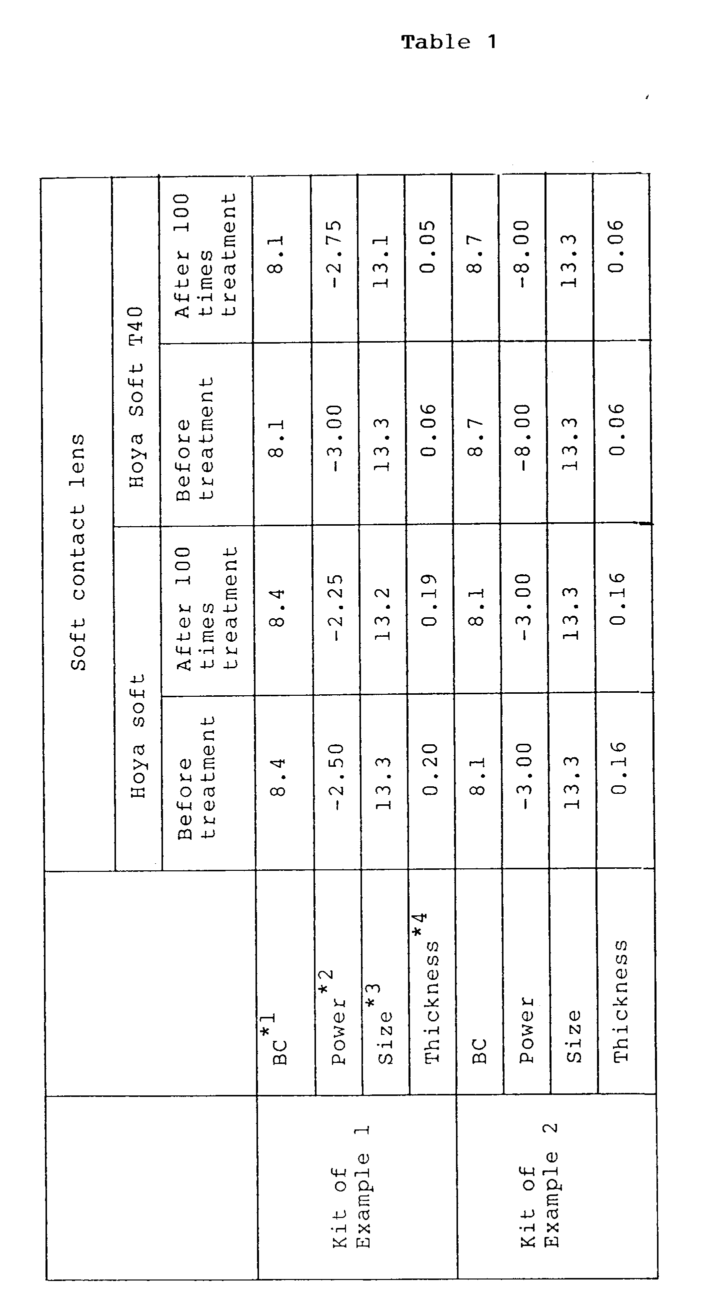

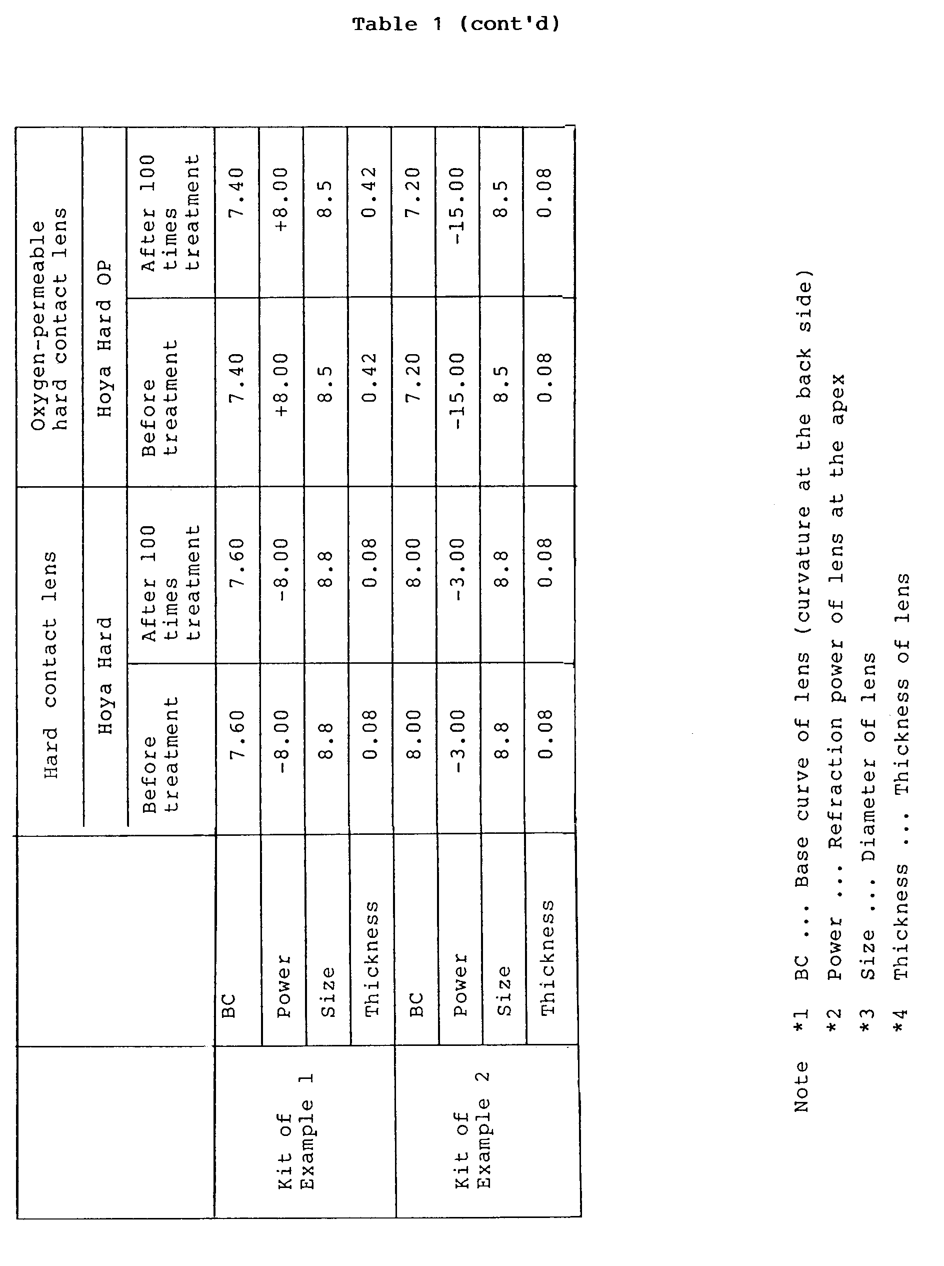

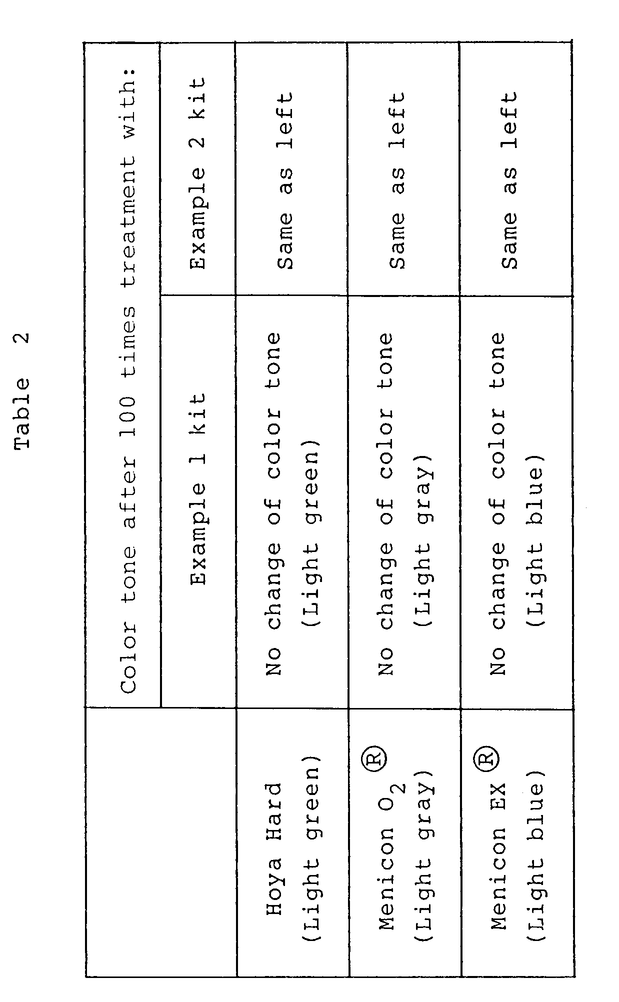

As obvious from Tables 1 and 2, in repeated cleaning of contact lenses using the kits of Examples 1 and 2, neither parameter changes not discoloration occurred in any lenses. Thus, it was confirmed that the package has no effect on the shape, etc. of the contact lens.

Next, the amount of available chlorine remaining in the solution after treatment when the kits of Examples 1 and 2 were used was measured in accordance with the color reaction by o-toluidine hydrochloride. In all cases, the solutions after treatment gave no color development indicating presence of residual available chlorine. In contrast, all of the treating solutions containing only powders (the oxidizing agent) and containing no tablets (the reducing agent) gave distinct color development. The solutions after treatment were dropped in eyes, but there was neither stimulus nor damage to the cornea.

Thus, it was confirmed that the treating solutions cause no damage to eyes and are safe.

## Claims

1. The use, for cleaning without discoloring a contact lens, of a package containing an oxidizing agent comprising chlorinated lime powder for stain-removal and a tablet, the contents of the tablet comprising (i) sodium or potassium bicarbonate and (ii) a reducing agent comprising citric acid or citric acid and sodium citrate, the tablet being so coated that the powder does not substantially react with the contents of the tablet in the package and that when the tablet and the powder are placed in water substantially simultaneously the major portion of the chlorinated lime dissolves more rapidly than the major portion of component (ii), whereby, on dissolution of the tablet coating in the water, effervescence takes place and residual chlorinated lime is reduced by component (ii) to render it nontoxic.

2. Use according to claim 1, wherein the package is a three sides-sealed aluminium package or a stick-shaped aluminium package.

3. Use according to claim 1 or claim 2, wherein the package contains a predetermined quantity of the oxidizing and reducing agents with instructions to dissolve the contents of the package in a specified quantity of water.

4. Use according to any one of claims 1 to 3, wherein the treating solution after the completion of cleaning is isotonic with and has the same pH as the human lacrima.

5. Use according to any one of claims 1 to 4, wherein the concentration of the oxidizing agent after dissolution in water is 0.005% to 5.0% by w/v.

6. Use according to any one of claims 1 to 5, wherein the concentration of the reducing agent after dissolution in water is 0.05% to 10.0% by w/v.

## Patentansprüche

1. Verwendung einer Packung mit einem chloriertes Kalkpulver umfassenden Oxidationsmittel zur Fleckenentfernung und einer Tablette, enthaltend
(i) Natrium- oder Kaliumbicarbonat und
(ii) ein Zitronensäure oder Zitronensäure und Natriumcitrat umfassendes Reduktionsmittel,
wobei die Tablette derart beschichtet ist, daß das Pulver mit dem Tabletteninhalt in der Packung praktisch nicht reagiert und daß im Falle, daß die Tablette und das Pulver praktisch gleichzeitig in Wasser eingebracht werden, sich der Hauptteil des chlorierten Kalks rascher löst als der Hauptteil der Komponente (ii), so daß es beim Auflösen des Tablettenüberzugs in dem Wasser zu einem Aufbrausen kommt und restlicher chlorierter Kalk durch die Komponente (ii) unter Beseitigung seiner Toxizität reduziert wird, zur Säuberung einer Kontaktlinse ohne Verfärbung.

2. Verwendung nach Anspruch 1, wobei die Packung aus einer dreiseitig versiegelten Aluminiumpackung oder einer stiftförmigen Aluminiumpackung besteht.

3. Verwendung nach Anspruch 1 oder 2, wobei die Packung eine gegebene Menge an Oxidations- und Reduktionsmitteln und Instruktionen zum Auflösen des Packungsinhalts in einer gegebenen Menge Wasser enthält.

4. Verwendung nach einem der Ansprüche 1 bis 3, wobei die Behandlungslösung nach Beendigung der Säuberung mit menschlicher Tränenflüssigkeit isotonisch ist und denselben pH-Wert wie menschliche Tränenflüssigkeit aufweist.

5. Verwendung nach einem der Ansprüche 1 bis 4, wobei die Konzentration des Oxidationsmittels nach dem Auflösen in Wasser 0,005 - 5,0% (g/v) beträgt.

6. Verwendung nach einem der Ansprüche 1 bis 5, wobei die Konzentration des Reduktionsmittels nach dem Auflösen in Wasser 0,05 - 10,0% (g/v) beträgt.

## Revendications

1. L'emploi, pour nettoyer des verres de contact sans en altérer la couleur, d'un emballage contenant un agent oxydant d'une poudre de chaux chlorée pour enlever les taches, avec un comprimé qui comprend (1) du bicarbonate de sodium ou de potassium et (2) un agent réducteur constitué d'acide citrique, seul ou avec du citrate de sodium, comprimé qui est enrobé pour que la poudre ne puisse pas réagir avec son contenu dans l'emballage et que quand le comprimé et la poudre sont mis en même temps dans de l'eau, la majeur partie de la chaux chlorée se dissolve plus rapidement que la majeur partie du composant (2), et ainsi, quand l'enrobage du comprimé se dissout dans l'eau, il y a effervescence et la chaux chlorée résiduelle est réduite par le composant (2), ce qui supprime sa toxicité.

2. Emploi selon la revendication 1 pour lequel l'emballage est un emballage en aluminium scellé sur trois côtés ou en forme de bâton.

3. Emploi selon la revendication 1 ou 2 pour lequel l'emballage contient des quantités déterminées de l'oxydant et du réducteur, avec des instructions pour dissoudre son contenu dans une quantité d'eau spécifiée.

4. Emploi selon l'une quelconque des revendications 1 à 3 pour lequel la solution de traitement, quand le nettoyage est achevé, est isotonique avec la sécrétion lacrymale et a le même pH.

5. Emploi selon l'une quelconque des revendications 1 à 4 pour lequel concentration de l'agent oxydant après dissolution dans l'eau est de 0,005 à 5,0 % en poids par volume.

6. Emploi selon l'une quelconque des revendications 1 à 5 pour lequel la concentration de l'agent réducteur après dissolution dans l'eau est de 0,05 % à 10,0 % en poids par volume.
